# EUROPÄISCHE PATENTANMELDUNG

(11) **EP 2 223 665 A1**
(43) Veröffentlichungstag der Anmeldung: **01.09.2010**
(21) Anmeldenummer: 10001765.6
(22) Anmeldetag: 22.02.2010
(51) Int. Cl.: A61B 17/80

(54) **Implantat zur Fusion von Knochen beziehungsweise Knochenteilen, insbesondere des Handwurzelbereichs eines Handgelenks**

(30) Priorität: 23.02.2009 DE 202009001900 U
(71) Anmelder: Zrinski AG, 78573 Wurmlingen (DE)
(72) Erfinder: Zrinski, Josef, 78573 Wurmlingen (DE); Hausam, Volker, 78259 Mühlhausen-Ehingen (DE); Eckhof, Stephan, 78604 Weilheim-Rietheim (DE)

(57) **Zusammenfassung**

Bei einem Implantat (1) zur Fusion von Knochen oder Knochenteilen (K1 bis K2), insbesondere des Handwurzelbereichs eines Handgelenks, sowie zum Aufbringen einer Kompression zwischen den Knochen oder Knochenteilen (K1 bis K4), ist ein Grundkörper (2) mit Knochenschrauben (6) vorgesehen, die in Ausnehmungen (5) geführt sind, die eine Geometrie besitzen, durch welche die Kompression zwischen den jeweiligen Knochen (K1 bis K4) einstellbar ist, wobei der Grundkörper (2) rotationssymmetrisch ausgebildet ist und die Geometrie seiner Ausnehmungen (5) jeweils die Form eines in Umfangsrichtung des Grundkörpers (2) gekrümmtes Langloches hat, dessen Krümmung bezogen auf die zentrale Achse (3) des Grundkörpers (2) derart zu dieser geneigt verläuft, dass die in die Knochen (K1 bis K4) eingeschraubten und in den Ausnehmungen (5) kulissenartig geführten Knochenschrauben (6) durch eine Drehbewegung (Pfeil 7) des Grundkörpers (2) in Richtung der geneigten Langlöcher radial zu der zentralen Achse (3) des Grundkörpers (2) hin bewegbar sind.

## Beschreibung

### Technisches Gebiet

Die Erfindung betrifft ein Implantat zur Fusion von Knochen bzw. Knochenteilen, insbesondere des Handwurzelbereichs eines Handgelenks, sowie zum Aufbringen einer Kompression zwischen den Knochen bzw. Knochenteilen, mit einem Grundkörper mit Knochenschrauben, die in Ausnehmungen geführt sind, die eine Geometrie aufweisen, durch welche die Kompression zwischen den Knochen bzw. Knochenteilen einstellbar ist. Die Größe des Implantats ist dabei so gewählt, dass sich dieses zumindest über eine Teil der Knochen bzw. Knochenteile erstreckt.

### Stand der Technik

Implantate der vorstehenden Art dienen zur Osteosynthese, insbesondere der gezielten Arthrodese von Teilen des Handgelenks. Als Indikation können hierfür unter anderem akute Frakturen im Handwurzelbereich, vor allem aber degenerative Arthritis in Folge veralteter Pseudoarthrosen des Os scaphoideums oder veralteten Rupturen der ligamentären Verbindung zwischen Os scaphoideum und Os lunatum vorliegen. Unbehandelt führen diese, begleitet von einer schmerzhaften Symptomatik, in der Regel zu einer permanenten Schädigung des Handgelenks.

Für die Übertragung von Muskelkräften und das Bewegen und Greifen von Gegenständen ist ein stabiles und bewegliches Handgelenk unbedingt erforderlich. Die Wiederherstellung einer schmerzfreien Belastung der Hand kann mit einer gewissen Bewegungseinschränkung und Verminderung der Greifkraft durch eine partielle Versteifung erzielt werden.

Im Handwurzelbereich sind in diesem Zusammenhang fünf Knochen von wesentlicher Bedeutung. Dies sind das Os scaphoideum, Os lunatum, Os triquetrum, Os capitatum und das Os hamatum.

Ein chirurgischer Eingriff sieht diesbezüglich die Resektion des Os scaphoideums und die Fusion der restlichen vier genannten Knochen vor. Hierzu werden nach dem Stand der Technik bisher im Wesentlichen so genannte Kirschner-Drähte und Osteosyntheseplatten zur Fixierung der Knochen und dem Herbeiführen einer Osteosynthese verwendet.

Eine Osteosynthese wird durch das Vorhandensein einer Kompression der sich berührenden Knochenflächen erheblich begünstigt. Das Aufbringen einer Kompressionskraft ist daher gemeinsames Ziel solcher chirurgischen Verfahren. Gelingt das Aufbringen einer ausreichenden Kompressionskraft nicht, so kann eine so genannte Pseudoarthrose die Folge sein. Das Ausbleiben der gewünschten Knochenbildung führt hierbei zu einem sehr schmerzhaften Zustand.

Eine Kompression zwischen Knochensegmenten kann z. B. auch durch das Einbringen autologer Knochensubstanz erfolgen. Die Gegenkraft zur interossären Kompression wird durch die umschließenden ligamentären Strukturen aufgebracht. Diese Prozedur ist mit einem zusätzlichen invasiven Eingreifen verbunden.

Die so hergestellte Kompression muß in jedem Fall stabilisiert werden, wofür aus der Technik unterschiedliche Verfahren bekannt sind. Eine Möglichkeit besteht darin, die bereits erwähnten Kirschnerdrähte windschief zueinander durch die zu fusionierenden Knochen einzubringen. Eine weitere Möglichkeit besteht darin, durch speziell geformte Platten die angestrebte Stabilität herbeizuführen.

Eines dieser Implantate ist ein scheibenförmiges Element, das auf seiner Fläche entsprechende Ausnehmungen aufweist, die zur Aufnahme von Befestigungsmitteln, insbesondere Knochenschrauben, geeignet sind. Diese Ausnehmungen sind derart ausgebildet, dass die Befestigungsmittel in einem variablen Winkel in diese einführbar sind, wodurch die Handwurzelknochen entsprechend erreicht werden können. Das Einschrauben der Knochenschrauben führt dabei zu einer Relativbewegung der gegriffenen Knochenelemente, und zwar zum Implantat hin. Aufgrund dessen, dass das Implantat vorzugsweise zum Knochen hin kegelförmig ausgebildet ist, wird damit versucht, die Knochenelemente zentrisch zu fixieren.

Derartige Implantate gibt es in unterschiedlichen Grössen. Um nun das Implantat plan zur Knochenoberfläche einsetzen zu können, wird die Kontur des Implantats in die Handwurzelknochen präpariert (Ausfräsen der entsprechenden Knochenteile).

Die Kompression, die erforderlich ist, um die Osteosynthese zu erreichen und um auch den Schmerz vom Patienten entsprechend zu mindern, ist aufgrund der bisherigen Operationstechniken nicht immer gegeben.

Hierzu ist aus dem Stand der Technik beispielsweise aus der US 7,201,752 B2 (22. Juli 2003; Acumed LLC**)** ein Verfahren sowie ein System bekannt, das zur Fusion von zwei oder mehreren Knochen eine Platte vorsieht, die scheibenartig und zur Knochenseite hin sphärisch ausgebildet ist. In der scheibenförmigen Ausbildung sind mehrere Bohrungen vorgesehen, die wahlweise von einem oder mehreren Knochenschrauben durchdrungen werden können. Für das Anbringen des Systems wird - wie bereits zuvor beschrieben -, der Handwurzelbereich entsprechend vorbereitet und eine Ausfräsung erstellt. Beim Einlegen dieser Vorrichtung in ein derartiges Knochenbett, kann diese dann so positioniert werden, dass zumindest eine Knochenschraube jeweils einen zu fusionierenden Knochen durchdringt. Die Knochenschrauben können dabei in unterschiedlichen Winkeln durch die Vorrichtung geführt werden. Durch das Einschrauben der nochenschrauben in die jeweiligen Knochenteile können diese sodann an die Platte herangezogen werden.

Aufgrund der Vielzahl der entsprechenden Bohrungen, die diese Vorrichtung vorsieht und die mögliche Anordnung der Knochenschrauben in die unterschiedlichen Winkelstellungen, kann hiermit eine relativ gute Fixierung erzielt werden, wobei diese Fixierung auch noch dadurch unterstützt wird, dass mittig eine entsprechende Schraube einbringbar ist.

Auch die US 6,179,839 B1 (20. September 1999; Weiss et al. ) zeigt eine Fusionsplatte, die geeignet ist, mehrere Knochenteile zusammen zu führen, indem eine Vielzahl von Bohrungen innerhalb der Vorrichtung vorgesehen sind, die von Knochenschrauben durchdrungen werden können. Aufgrund der kegelförmigen Ausbildung zur Knochenseite hin, können durch das Einschrauben der Knochenschrauben in die entsprechenden Knochenteile diese zentrisch an der Fusionsplatte fixiert werden.

Ferner ist aus der DE 20 2008 005 076 U1 **11.04.2008, Zrinski AG)** ein Implantat zur Fusion von Knochen bzw. Knochenteilen bekannt, das im Wesentlichen aus mehreren Gleitsteinelementen besteht, die innerhalb des Implantats beweglich gelagert sind. Die Gleitsteinelemente dienen dabei zusätzlich zur Aufnahme von Knochenschrauben. Ferner ist ein Kraftelement vorgesehen, das derart ausgebildet ist, dass dieses die Gleitsteinelemente jeweils mit einer Kraft F beaufschlagt und diese dadurch bewegen kann. Für das Anbringen des Implantats ist vorgesehen, dieses auf die entsprechenden zu fusionierenden Knochen aufzulegen; sodann die Knochenschrauben zu platzieren und anschliessend ein im Zentrum des Implantats vorgesehenes Stellelement derart zu bewegen, dass dadurch die Beweglichkeit der Gleitsteine freigegeben wird. Aufgrund der aufgebrachten Kräfte F bewegen sich die Gleitsteine zentrisch zum Implantatmittelpunkt hin und komprimieren damit gleichzeitig die zu fusionierenden Knochen.

Schließlich ist in der EP 0 016 338 A1 (15.02.1980, Prof. Dr. Brinckmann**)** eine Spannvorrichtung für eine Druckplatte zu Durchführung einer Druckosteosynthese beschrieben, mit der Spannweiten von 12 - 15 mm zwischen zusammenbringbaren Knochenfragmenten realisierbar sein sollen.

Hierbei besitzt die Druckplatte eine so genannte Fixationsseite und eine Spannseite, von denen die Fixationseite als erstes mit einem der Knochenfragmente mittels Knochenschrauben verbunden wird. Auf der Spannseite dagegen ist in der Druckplatte ein Langloch vorgesehen, über das ein Spannblock gesetzt werden kann, der an der Druckplatte mittels einer Schraube festlegbar ist. Innerhalb des Spannblock wiederum ist ein ovales Schraubenloch vorgesehen, das eine ovale Randneigung mit einer etwa 45° Neigung als eine Art schiefe Ebene besitzt. Mit dieser Randneigung des Schraubenloches arbeitet dann eine als Knochenschraube ausgebildete Spannschraube zusammen, die einen konischen Schraubenkopf aufweist.

Unter Zuhilfenahme dieser Spannschraube, können nun die beiden zu fusionierende Knochenfragmente unter erheblicher Kompressionsspannung aufeinander zubewegt werden, indem die Spannschraube durch deren Drehung entlang der ovalen Randneigung gleitet, die ja eine schiefe Ebene darstellt und dadurch die gewünschte Vorschubbewegung der Knochenfragmente bewirkt.

Nachdem eine ausreichende Kompressionsspannung zwischen den zu verbindenden Knochenfragmenten erreicht worden ist, wird sodann die erhaltene Stellung der Druckplatte durch das Einschrauben einer Befestigungsknochenschraube auf der Spannseite der Druckplatte festgelegt und danach der Spannblock wieder entfernt.

### Nachteile des Standes der Technik

Insbesondere die Techniken, die aus dem Stand der Technik im Zusammenhang mit Implantaten für Handgelenke bekannt sind, weisen gemeinsam den Nachteil auf, dass die damit zu erhaltende Kompression der Knochen nicht ausreicht ist, um ein befriedigendes Ergebnis zu erzielen.

Zudem werden Implantate verwendet, die kegelförmige Ausfräsungen im Bereich der zu fusionierenden Knochen erfordern, was jedoch den Querschnitt der entsprechenden Knochen schwächt. Dadurch kann eine zusätzliche Bruchgefahr, insbesondere dann, wenn die entsprechenden Knochenschrauben eingedreht werden, entstehen.

Ferner sind die entsprechenden Geometrien, die aus dem Stand der Technik bekannt sind um eine entsprechende Knochenfusion mittels einer Knochenplatte zu erreichen, relativ komplex, da sie fertigungstechnisch aufwendig und mit entsprechend vielen Bauteilen versehen sind.

Schließlich zeigt die Anwendung mit der aufgezeigten Druckplatte zwar eine Lösung, um zwei Knochenfragmente zu fusionieren, jedoch kann diese Technik nicht ohne weiters auf Implantate bei Handgelenken übertragen werden, da der Einsatz von mehreren separaten Spannblöcken schon allein aus Platzgründen nicht praktikabel ist-

### Aufgabe der Erfindung

Aufgabe der Erfindung ist es daher, ein Implantat zur Fusion von Knochen oder Knochenteilen, insbesondere des Handwurzelbereichs eines Handgelenks, bereit zu stellen, mit der Wirkung, dass eine im Vergleich zum Stand der Technik eine einstellbare und gleichzeitig eine einfacher zu erhaltende Kompression zwischen den Knochen bzw. Knochenteilen möglich ist.

### Lösung der Aufgabe

Diese Aufgabe wird erfindungsgemäß dadurch gelöst, das der Grundkörper des Implantats rotationssymmetrisch ausgebildet ist und die Geometrie seiner Ausnehmungen jeweils die Form eines in Umfangsrichtung des Grundkörpers gekrümmtes Langloches hat, dessen Krümmung bezogen auf das Zentrum des Grundkörpers derart zu diesem geneigt verläuft, dass die in die Knochen eingeschraubten und in den Ausnehmungen kulissenartig geführten Knochenschrauben durch eine Drehbewegung des Grundköpers in Richtung der geneigten Langlöcher radial zum Zentrum des Grundkörpers hin bewegbar sind.

### Vorteile der Erfindung

Einer der wesentlichen Vorteile besteht darin, dass das erfindungsgemässe Implantat konstruktiv einfach aufgebaut ist und dennoch damit gezielt Zugkräfte für eine verbesserte Kompression von zu verbindenden Knochenfragmente erhalten werden, was vor allem die zum Zentrum gekrümmt verlaufenden Langlöcher bewirken, in denen die Knochenschrauben kulissenartig quasi zwangsgeführt sind.

Mit der Auflage des erfindungsgemäßen Implantats auf zu fusionierende Knochteile werden durch ein Eindrehen der Knochenschrauben mit einer entsprechenden Winkelrichtung die Knochenteile erfasst und fixiert. Durch eine nachfolgende auf das Implantat ausgeübte Drehbewegung in Umfangsrichtung, werden die Schrauben, die in die Knochenteile eingedreht worden sind, kulissenartig in den Ausnehmungen des Implantats derart geführt, dass durch diese Drehbewegung die Knochenteile zwangsgeführt zentrisch zur zentralen Achse des Implantats hin bewegt werden. Dadurch können auch die möglichen Spalte, die zwischen den einzelnen Knochenteilen bestehen, verringert bzw. aufgehoben werden, und zwar bis zu dem Punkt, an dem eine ausreichende Kompression erreicht worden ist..

Um diese komprimierte Position zu fixieren, kann vorzugsweise vorgesehen werden, dass in dem nun durch die Drehung frei gewordenen Bereiche der jeweiligen als Langloch ausgebildeten Ausnehmungen weitere Knochenschrauben einzusetzen , wodurch es möglich ist, dass jedes Knochenteil mit mindestens zwei Schrauben fixiert ist.

Aufgrund des Einbringens mindestens einer weiteren Knochenschraube in den jeweiligen Restraum der Ausnehmungen kann daher verhindert werden, dass ein ungewolltes Zurückdrehen und damit eine Dekompression der Knochenteile erfolgt, wodurch auf sehr einfache Art und Weise eine Fusion der entsprechenden Knochenteile beschleunigt werden kann.

In weiterer vorteilhaften Ausgestaltungen des erfindungsgemässen Implantates kann vorgesehen sein, dass die Ausnehmungen des Grundkörpers jeweils durch einen Randbereich begrenzt sind, der eine sphärisch ausgebildete Oberfläche besitzt, die einem Teil eines Mantels einer Kugel entspricht, wodurch das Gleiten der Knochenschrauben begünstigt wird.

Ferner ist es besonders zweckmäßig, wenn der Grundkörper im Gegensatz zum Stand der Technik an seiner Unter- und Oberseite eben, d. h. plattenartig ausgebildet ist, wodurch irgendwelche vorherigen Ausfräsungen der zu verbindenden Knochenteile entfallen.

Auch ist es zweckmäßig, im Zentrum des Grundkörpers eine Schlüsselelement zum Drehen des Implantats in oder gegen den Uhrzeigersinn vorzusehen. Hierbei kann es sich um eine zentrisch angeordnete Aussparung handeln, die ein formschlüssiges Werkzeug aufnehmen und mit dem dann das Implantat in eine entsprechende Drehbewegung versetzt werden kann. Alternativ kann hierzu vorgesehen sein, dass auf dem Umfang des Implantats Aussparungen vorhanden sind, die mit einem Werkzeug korrespondieren, sodass mit Hilfe dieses Werkzeuges die entsprechende Drehung ausgeführt wird.

Die Ausbildung der langlochförmigen Ausnehmungen ist in anderen Worten derart vorgesehen, dass sich deren Krümmung, gemessen von der zentralen Achse zur Mittellinie jeder Ausnehmung, in Umfangsrichtung des Implantats sich kontinuierlich verringert. Dadurch wird erreicht, dass durch eine Drehung des entsprechenden Implantats die Knochenschrauben zur zentralen Achse hin bewegt werden und dadurch wiederum eine entsprechende Kompression der Knochenteile erzielt wird. Dies bedeutet weiter, dass die jeweiligen Krümmungen der Ausnehmungen nicht parallel zur Aussenkontur des rotationssymmetrischen Implantats verlaufen.

Alternativ hierzu kann die jeweilige Krümmung der langlochförmigen Ausnehmungen auch derart beschrieben werden, dass diese einen Radius aufweist, dessen Ursprungspunkt versetzt zur zentralen Achse angeordnet ist. Werden die bei diesem Beispiel vorliegenden vier Ursprungspunkte miteinander verbunden, entsteht ein Viereck.

Es ist auch möglich, dass der Verlauf der Krümmung der langlochförmigen Ausnehmungen im zur zentralen Achse nahen Bereich auf eine zu dieser Achse konzentrischen Bahn oder auf eine sich geringfügig von der zentralen Achse entfernenden Bahn schwenkt. Dadurch ist ein ungewolltes rotatorisches Lösen der aufgebrachten Kompression blockiert. Dadurch werden aber auch die zu komprimierenden Knochenteile zunächst aneinander geführt und anschliessend wieder geringfügig von einander wegbewegt. Was bedeutet, dass der Krümmungsradius jeder Ausnehmung sich im nahen Bereich zur zentralen Achse ändert.

Ferner ist es möglich, dass die Kompression nicht nur tangential erfolgt, sondern auch senkrecht. Dies kann dadurch erreicht werden, dass die Kulissenführung jeder Knochenschraube innerhalb der Ausnehmung derart ausgebildet wird, dass die Knochenschraube durch Drehung des Implantats geringfügig angehoben wird. Dadurch kann auch erreicht werden, dass in einer dreidimensionaler Ebene eine entsprechende Reponierung erzielbar ist.

Weitere vorteilhafte Ausgestaltungen gehen aus der nachfolgenden Beschreibung in Verbindungmit der Zeichnung und den Ansprüchen hervor.

### Zeichnungen

### Es zeigen:

- Fig. 1: eine perspektivische Ansicht auf das erfindungsgemässe Implantat, mit eingestzten Knochenschrauben;
- Fig. 2: eine perspektivische Ansicht auf den Grundkörper des Implantats gemäß Fig. 1, in einem kleineren Maßstab und ohne Knochenschrauben;
- Fig. 3: eine Draufsicht auf den Grundkörper gemäss Fig. 2;
- Fig. 4: eine Seitenansicht des in Fig. 2 dargestellten Grundkörpers;
- Fig. 5: einen Querschnitt durch den Grundköper gemäss Fig. 2, jedoch in einem größeren Maßstab;
- Fig. 6: eine Draufsicht zur Erläuterung der Funktionsweise eines an vier schematisch dargestellten Knochensegmenten fixierten Implantats in Fixierstellung;
- Fig. 7: die Draufsicht gemäss Fig. 6, jedoch ist hier der Grundkörper gegenüber Fig. 6 um 45 Grad im Uhrzeigersinn relativ zu den Knochensegmenten verdreht dargestellt;
- Fig. 8: die Draufsicht gemäss Fig. 7, jedoch mit zusätzlichen Knochenschrauben zur Vermeidung einer Dekompression, d. h. als Rotatationssicherung des Implantats in bereits komprimierter Stellung gemäss Fig. 7.

### Beschreibung eines Ausführungsbeispiels

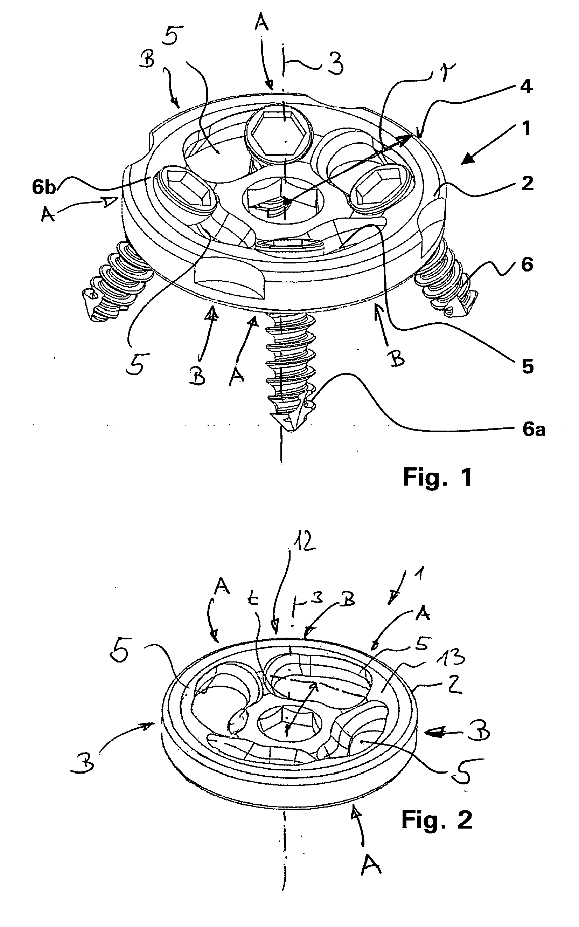

In den Fig. 1 - 5 ist ein erfindungsgemässes Implantat 1 dargestellt, , welches einen Grundkörper 2 besitzt, der als plattenförmiges Element ausgebildet ist. Bei dem hier dargestellten Ausführungsbeispiel handelt es sich um ein kreisrundes, d. h. rotationssymmetrisches Plattenelement mit einer zentralen Achse 3. Der Radius des Grundkörpers 2 ist durch den mit r bezeichneten Abstand bestimmt, der sich von der zentralen Achse 3 bis zum Aussenumfang 4 des Grundkörpers 2 erstreckt.

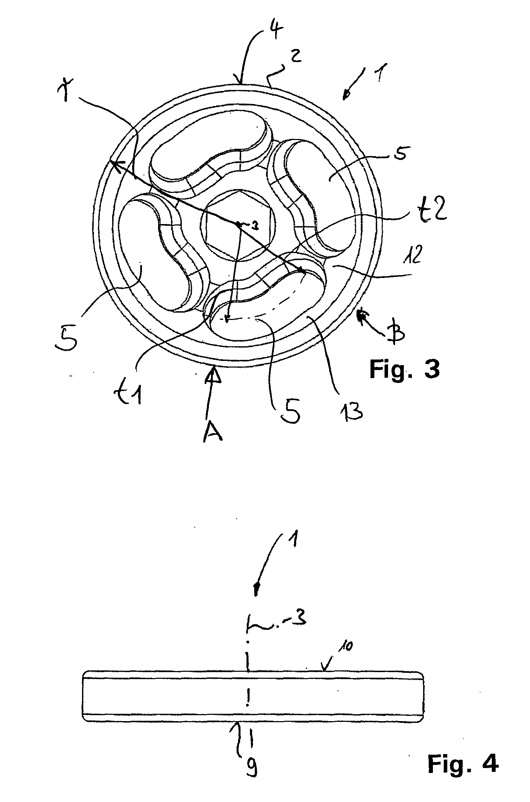

Der Grundkörper 2 besitzt vier Ausnehmungen 5, die langlochartig ausgebildet sind. Die Ausnehmungen 5 weisen wiederum eine Krümmung auf, die derart ausgebildet ist, dass sich der in Fig. 2 mit t bezeichnete Radius von der einen Seite A der jeweiligen langlochartigen Ausnehmung 5 bis zur deren anderen Seite B ändert, und zwar ausgehend vom Ursprungspunkt auf der zentralen Achse 3. Bevorzugt kann sich dieser Radius t so verringern, sodass eine nierenartige Form der Ausnehmungen 5 entsteht. In Fig. 3 ist dargestellt, dass ein Radius t1 am Ausgangspunkt (eine Seite A) grösser ist als ein Radius t2 am Endpunkt (andere Seite B).

Wesentlich ist vor allem, dass die in Umfangsrichtung des Grundkörpers 2 verlaufende Krümmung jeder Ausnehmung 5, bezogen auf die zentrale Achse 3 des Grundkörpers 2, derart zu dieser geneigt verläuft, dass jede in den Ausnehmungen 5 kulissenartig geführte Knochenschraube -bezeichnet mit 6- durch eine Drehbewegung des Grundkörper 2 in Richtung der geneigten Ausnehmungen 5 in der Form eines Langloches radial zur zentralen Achse 3 des Grundkörpers 2 hin bewegbar ist, wodurch dabei eine jeweilige Zugkraft auf den jeweiligen Knochen in Richtung auf das Zentrum des Grundkörpers 2 ausgeübt wird.

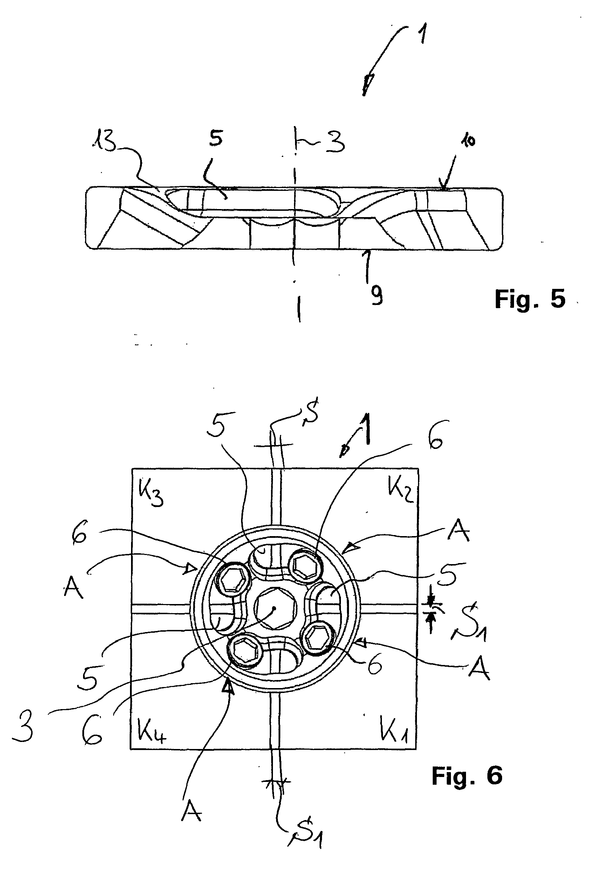

Gemäss den Fig. 4 und 5 ist das Implantat 1 scheibenförmig ausgebildet. Dies hat zur Folge, dass die zur Knochenseite hinweisende Seite - bezeichnet als Unterseite 9 - eben, d. h. plan ausgebildet ist, sodass keine kegelförmige oder sphärische Aufnahmeöffnung , sondern lediglich ein ebene Vertiefung innerhalb der Knochen vorhanden sein muss. Um eine plane Anlage zu ermöglichen, ist die von der Knochenseite abweisenden Seite -bezeichnet als Oberseite 10 - ebenfalls plan ausgebildet.

Die Ausnehmungen 5 des Implantats 1 weisen ferner einen Randbereich 12 auf, der sphärisch ausgebildet ist. Dadurch wird eine Oberfläche 13 geschaffen, die einem Teil eines Mantels einer Kugel entspricht. In die Ausnehmungen 5 sind die Knochenschrauben 6 zu stecken, welche aus einem Gewindeschaft 6 a und einem Kopfelement 6 b bestehen. Das Kopfelement 6 b ist dabei derart bemessen, dass dieses grösser ist als die Lichteweite der jeweiligen Ausnehmung 5. Aufgrund dessen, dass die Ausnehmungen 5 durch eine sphärische Oberfläche 13 begrenzt sind, erstrecken sich die Knochenschrauben 6 nicht senkrecht zum Implantat 1 und damit parallel zur zentralen Achse 3, sondern erstrecken sich senkrecht von der sphärischen Oberfläche 13 weg. Dadurch weisen die Knochenschrauben 6 nach aussen und sind damit geeignet, die einzelnen Knochenteile aufzunehmen.

### Funktionsweise:

### Die Funktionsweise wird nachfolgend anhand der Fig. 6 bis 8 beschrieben:.

Nachdem in die vier Knochenteile -bezeichnet mit K1, K2, K3 und K4- eine ebene Aufnahmeöffnung eingearbeitet worden ist, wird das Implantat 1 aufgelegt, und zwar derart, dass die Ausnehmungen 5 jeweils ein Knochenteil abdecken. Die Knochenteile K1, K2, K3 und K4 weisen untereinander einen Spalt S1 auf.

Anschiessend erfolgt die Fixierung, indem die Knochenschrauben 6 derart in die Knochenteile K1 bis K4 eingedreht werden, dass diese im Bereich der einen Seite A der langlochartigen Ausnehmungen 5 angeordnet sind.

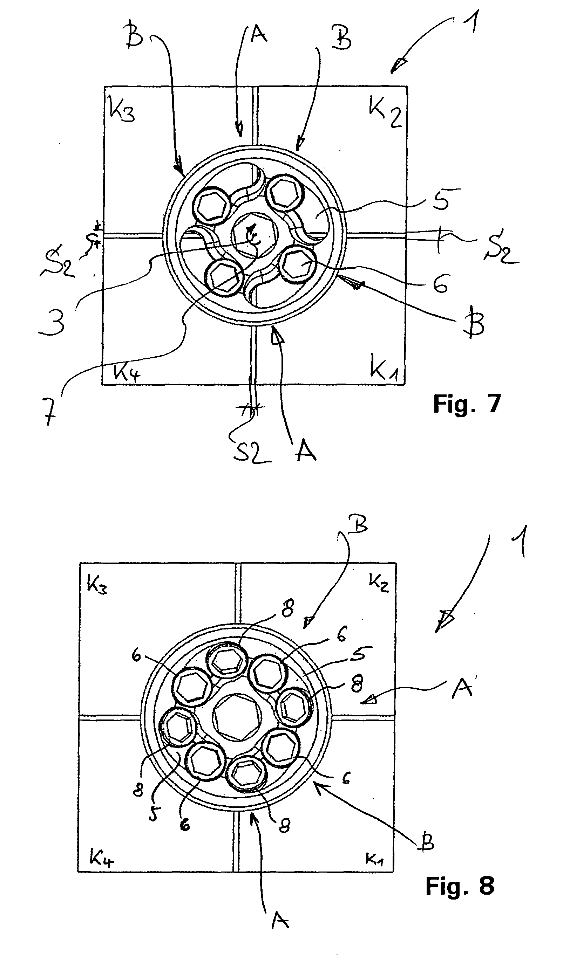

Durch ein anschließendes Verdrehen des Implantats 1 in Pfeilrichtung 7 (Fig. 7) wird bewirkt, dass die Knochenschrauben 6 von der einen Seite A an das andere Ende B der Ausnehmungen 5 gelangen. Da die Knochenschrauben 6 die jeweiligen Knochenteile K1 bis K4 fixieren und die Ausnehmungen 5 eine Krümmung aufweisen, die zur zentrischen Achse 3 hinweist, werden die Knochenteile K1 bis K4 gleichzeitig zentrisch zur Achse 3 hingeführt. Dadurch wird wiederum der Spalt S1 gemäss Fig. 6 kleiner bzw. aufgehoben, wobei der kleinere Spalt mit dem Bezugszeichen S2 in Fig. 7 bezeichnet ist..

Um zu verhindern, dass sich die fixierte Stellung, wie sie in Fig. 7 dargestellt ist, ungewollt löst, werden bei dem hier dargestellten Ausführungsbeispiel zusätzlich in den vorhandenen Räumen der Ausnehmungen 5 (im Bereich des einen Endes A) jeweils mindestens eine weitere Knochenschraube 8 platziert. Bei diesem Ausführungsbeispiel durchdringen die Knochenschrauben 8 ebenfalls die Ausnehmungen 5 und durchdringen ebenfalls zumindest zum Teil die Knochenteile K1 bis K4. Alternativ dazu kann auch vorgesehen sein, dass anstelle der Knochenschrauben 8 ausschliesslich Kopfschrauben verwendet werden, die nur in das Implantat 1 eingeschraubt werden, um zu verhindern, dass das die Knochenschrauben 6 sich nicht innerhalb der Ausnehmungen 5 bewegen können.

Mit dem erfindungsgemässen Implantat 1 ist eine medizinische Vorrichtung geschaffen worden, die auf einfache Art und Weise eingesetzt werden kann und mit deren Hilfe eine ausreichende Kompression der einzelnen zu fusionierenden Knochenteile erreicht werden kann. Durch einfaches Fixieren der entsprechenden Knochenteile K1 bis K4 sowie ein Verdrehen des Implantats 1 um einen definierten Winkel, werden die einzelnen aufgenommen Knochenteile K1 bis K4 soweit aneinander herangeführt, dass eine Kompression erreicht wird. Zusätzliche Mittel, wie beispielsweise Kopfschrauben oder weitere Knochenschrauben dienen dazu, ein Wiederaufdrehen bzw. eine Dekompression zu verhindern. Alternativ kann diese Sicherung durch einen geeigneten Krümmungs- bzw. Kulissenverlauf der Schrauben aufnehmenden Ausnehmungen 5 erfolgen.

Das Implantat 1 lässt sich einfach fertigen, da es eben ausgebildet ist und eine einfache Form der entsprechenden Ausnehmungen 5 zur Aufnahme der entsprechenden Knochenschrauben 6 bzw. 8 aufweist. Weiterhin begrenzt sich die Anzahl der Komponenten auf das eigentliche Implantat 1 mit den Knochenschrauben 6 bzw. 8 als Befestigungsmittel.

### Bezugsteileliste

- 1: Impantat
- 2: Grundkörper
- 3: Zentrum (Achse)
- 4: Außenumfang
- 5: Ausnehmung
- 6: Knochenschraube
- 6a: Gewindeschaft
- 6b: Kopfelement
- 7: Pfeilrichtung
- 8: Knochenschraube
- 9: Unterseite
- 10: Oberseite
- 11: ---
- 12: Randbereich
- 13: sphärische Oberfläche

- A-Seite:
- B-Seite:
- K1 bis K4: Knochenteile
- r: Radius
- t1: Radius
- t2: Radius
- S1: Spalt
- S2: verkleinerter Spalt

## Patentansprüche

1. Implantat (1) zur Fusion von Knochen oder Knochenteilen (K1 bis K4), insbesondere des Handwurzelbereichs eines Handgelenks, sowie zum Aufbringen einer Kompression zwischen den Knochen oder Knochenteilen (K1 bis K4), mit einem Grundkörper (2) mit Knochenschrauben (6), die in Ausnehmungen (5) geführt sind, die eine Geometrie aufweisen, durch welche die Kompression zwischen den jeweiligen Knochen (K1 bis K4) einstellbar ist, **dadurch gekennzeichnet, dass** der Grundkörper (2) rotationssymmetrisch ausgebildet ist und die Geometrie seiner Ausnehmungen (5) jeweils die Form eines in Umfangsrichtung des Grundkörpers (2) gekrümmten Langloches hat, dessen Krümmung bezogen auf die zentrale Achse (3) des Grundkörpers (2) derart zu dieser geneigt verläuft, dass die in die Knochen (K1 bis K4) eingeschraubten und in den Ausnehmungen (5) kulissenartig geführten Knochenschrauben (6) durch eine Drehbewegung (Pfeilrichtung 7) des Grundkörpers (2) in Richtung der geneigten Langlöcher radial zur zentralen Achse (3) des Grundkörpers (2) hin bewegbar sind.

2. Implantat nach Anspruch 1, **dadurch gekennzeichnet, dass** die Ausnehmungen (5) des Grundkörpers (2) jeweils durch einen Randbereich (12) begrenzt sind, der eine sphärisch ausgebildete Oberfläche (13) aufweist, die einem Teil eines Mantels einer Kugel entspricht.

3. Implantat nach Anspruch 1 oder 2, **dadurch gekennzeichnet, dass** der Grundkörper (2) auf seiner Unterseite (9) und/oder Oberseite (10) eben ausgebildet ist.

4. Implantat nach einem der vorherigen Ansprüche, **dadurch gekennzeichnet, dass** das Zentrum des Grundkörpers (2) zur Aufnahme eines formschlüssigen Drehwerkzeugs eine entsprechende Aussparung aufweist.

5. Implantat nach mindestens einem der vorherigen Ansprüche, **dadurch gekennzeichnet, dass** zur Vermeidung einer Dekompression zusätzlich weitere Knochenschrauben (8) oder Kopfschrauben in dem Restraum der Ausnehmungen vorgesehen sind.
